# EUROPÄISCHE PATENTANMELDUNG

(11) **EP 2 322 145 A1**
(43) Veröffentlichungstag der Anmeldung: **18.05.2011**
(21) Anmeldenummer: 10013702.5
(22) Anmeldetag: 15.10.2010
(51) Int. Cl.: A61K 9/48, A61K 36/15, A61K 36/14

(54) **Stoffgemisch auf Basis einer Mischung von ätherischen Ölen und Verwendung**

(30) Priorität: 13.11.2009 DE 102009053089
(71) Anmelder: Brøndlund, Marianne, 8462 Harlev (DK)
(72) Erfinder: Brøndlund, Marianne, 8462 Harlev (DK)
(74) Vertreter: Laufhütte, Dieter

(57) **Zusammenfassung**

Die Erfindung betrifft Stoffgemisch auf Basis einer Mischung von ätherischen Ölen, wobei die Öl-Mischung Wacholderöl und Terpentinöl, insbesondere Terpentinöl vom Strandkiefer-Typ enthält oder im Wesentlichen daraus besteht, und wobei das Stoffgemisch vorzugsweise des Weiteren einen oder eine Mischung mehrerer flüssiger und/oder gelöster Wirkstoffe und/oder einen oder eine Mischung mehrerer pulverförmiger Wirkstoffe beinhaltet.

## Beschreibung

Die vorliegende Erfindung betrifft ein Stoffgemisch auf Basis einer Mischung von ätherischen Ölen sowie die Verwendung eines Stoffgemisches auf Basis einer Mischung von ätherischen Ölen.

Der Einsatz ätherischer Öle in pharmazeutischen, insbesondere pflanzlich pharmazeutischen Mitteln ist im Stand der Technik bekannt. So offenbart die DE 39 02 981 A1 den Einsatz von ätherischen Ölen gemeinsam mit verschiedenen Trägerstoffen auf einem Pflaster. Dabei sollen Wirkstoffe für die transdermale Therapie bereitgestellt werden. In der DE 699 37 692 T2 wird die Verwendung von ätherischen Ölen in Kombination mit 1-Menthol als dermaler Wirkstoff mit Migräne mindernder Wirkung vorgeschlagen. Als bevorzugte Präparationen werden Salben und Pflaster genannt. Die EP 0 800 827 B1 offenbart die medizinische Verwendung von ätherischen Ölen zur Regelung der Blutplättchenaggregation, alleine oder in Verbindung zu einer Behandlung mit Acetylsalicylsäure. Blutplättchenaggregation kann beispielsweise durch Adrenalinausschüttung oder durch das Rauchen hervorgerufen werden, wobei mit ätherischen Ölen insbesondere bei der adrenalininitiierten Blutplättchenaggregation ein über die bloße Therapie mit AAS hinausgehender Behandlungserfolg erreicht werden soll.

Während im Stand der Technik wie eben diskutiert vor allem die dermale Anwendung von ätherischen Ölen betont wird, offenbart die DE 698 30 049 T2 eine medizinische oder kosmetische Zusammensetzung auf Basis ätherischer Öle, wobei derartige medizinische Zusammensetzungen zur oralen Einnahme geeignet sind.

In den letzten Jahrzehnten ließ sich allgemein eine gesteigerte Nachfrage nach pflanzenbasierten und natürlichen Arzneimitteln, oft als Ergänzung zur konventionellen Behandlung verzeichnen. Solche Arzneimittel sind häufig Nebenwirkungsarm und stehen im Einklang mit einer naturbewussten Lebensweise, was u. a. für ihre steigende Beliebtheit unter Patienten verantwortlich ist. Während der Einsatz von einzelnen ätherischen Ölen in pflanzlichen Arzneimitteln, insbesondere topisch wirksamen pflanzlichen Arzneimitteln bekannt ist, ist es nun ein Ziel der vorliegenden Erfindung, verschiedene ätherische Öle in oral einnehmbaren Arzneien zu kombinieren und durch diese Kombination der darin enthaltenen pflanzlichen Wirkstoffe eine Basis für neue pflanzliche Arzneien bereitzustellen. Damit sollen gegebenenfalls in Kombination mit weiteren medikamentösen Therapien Behandlungserfolge bei Infektionskrankheiten, Husten, Erkältung, Bronchitis, Nebenhöhlenentzündung, allergischen Reaktionen, Asthma, Krankheitsbildern des Herz-Kreislaufsystems, Miktionsstörungen bei Prostatahyperplasie, Wundheilungsprobleme, Kieferprobleme, Zahnschmerzen, Rheuma und Bauchschmerzen erreicht werden.

Durch den wohltuenden Geruch, die wärmenden Eigenschaften und die allgemein sinnesreizenden Eigenschaften der ätherischen Öle soll die Genesung dabei auf physischer und psychischer Ebene gestützt werden. Insbesondere soll diese Mischung von ätherischen Ölen als Basis für den Zusatz mehrerer anderer pharmazeutischer Wirkstoffe, insbesondere pflanzlicher Wirkstoffe und homöopathischer Stoffe dienen.

Dieses Ziel wird durch ein Stoffgemisch nach Anspruch 1 erreicht. Vorteilhafte Ausgestaltungen ergeben sich aus den Unteransprüchen.

Demnach ist erfindungsgemäß ein Stoffgemisch auf Basis einer Mischung von ätherischen Ölen vorgesehen, wobei die Öl-Mischung Wacholderöl und Terpentinöl, insbesondere Terpentinöl vom Strandkiefer-Typ enthält. In einer anderen Ausführungsform besteht die Öl-Mischung im Wesentlichen aus Wacholderöl und Terpentinöl, insbesondere Terpentinöl vom Strandkiefer-Typ.

Wacholderöl trägt den lateinischen Namen Oleum Juniperi e Baccarae, Terpentinöl vom Strandkiefer-Typ Oleum Terebinthinae rectificat.

Eine nichtsynthetische Arznei oder ein nichtsynthetisches Nahrungsergänzungsmittel auf Basis der Kombination dieser zwei ätherischen Öle kann sich insbesondere zur alternativen oder ergänzenden Behandlung von Infektionen, durch allergische Reaktion bedingten Symptomatiken wie Asthma oder Krankheitsbildern des Herz-Kreislaufsystems, Miktionsstörungen bei Prostatahyperplasie, Wundheilungsprobleme, Kieferprobleme, Zahnschmerzen, Rheuma und Bauchschmerzen eignen. Dabei ist eine Wirkung bei Erkältungsbeschwerden wie Husten, Schnupfen, Nasennebenhölenentzündung, Erkältungskopfschmerz, Bronchitis, Lungenentzündung u.ä. besonders hervorzuheben. Gegebenenfalls kann ein Stoffgemisch auf Basis dieser Mischung aus Wacholderöl und Terpentinöl gemeinsame mit einem Grippeprogramm aus dem Stand der Technik verwendet werden.

Das Stoffgemisch ist des Weiteren durch die Schnelligkeit und die Stärke des Wirkungseintrittes als Arzneimittel zum Einsatz bei Grippe, gegebenenfalls einer Erkrankung im Zusammenhang mit einer Grippe-Epidemie, speziell geeignet. Inflammationseffekte werden stark minimiert, sodass auch die unangenehmen und potentiell bedrohlichen, starken Symptome bei akut infizierten Patienten rasch gelindert werden können.

Die Konzentration der Inhaltsstoffe eines erfindungsgemäßen Stoffgemisches kann so stark gewählt werden, dass sich die Krankheitsdauer deutlich reduziert. Nähere Konzentrationsangaben werden im Zusammenhang mit nachfolgenden Ausführungsformen diskutiert. Sobald der Körper beschwerdefrei ist, sollte die Einnahme des erfindungsgemäßen Stoffgemisches, optional zusammen mit dem Grippeprogramm aus dem Stand der Technik eingestellt werden.

Vorzugsweise beinhaltet ein erfindungsgemäßes Stoffgemisch auf Basis einer Mischung von ätherischen Ölen des Weiteren einen oder eine Mischung mehrerer flüssiger oder gelöster Wirkstoffe und/oder einen oder eine Mischung mehrerer pulverförmiger Wirkstoffe. Dadurch können verschiedenste Wirkstoffe und Hilfsstoffe in das erfindungsgemäße Stoffgemisch miteingebracht werden, wodurch die Wirkung des Öl-Gemischs verstärkt werden kann oder andere verschiedenste Wirkungen erfindungsgemäßer Stoffgemische erst hervorgerufen werden können.

In einer bevorzugten Ausführungsform ist ein erfindungsgemäßes Stoffgemisch zur oralen Einnahme bestimmt, insbesondere als Nahrungsergänzungsmittel. Die Wirkung des erfindungsgemäßen Stoffgemisches soll sich bei der oralen Einnahme entfalten.

In einer weiteren bevorzugten Ausführungsform liegt das erfindungsgemäße Stoffgemisch portioniert in Form einer oral einnehmbaren Kapsel, vorzugsweise einer Weichgelatinekapsel vor. Durch den Einbau in eine Kapsel kann sichergestellt werden, dass die gewünschte Dosierung exakt eingehalten wird, dass das Stoffgemisch vom Patienten mit gewünschter geschmacklicher oder geruchlicher Stärke einnehmbar ist, was insbesondere durch die intensiven organoleptischen Eigenschaften von Wacholderöl und Terpentinöl wünschenswert ist, und/oder dass das ungehemmte Eindringen hochkonzentrierter Wacholder- oder Kieferextrakte in die Atemwege geregelt, verringert oder verhindert wird. Eine Weichgelatinekapsel ist deshalb besonders vorteilhaft, da herkömmliche Plastikkapseln durch den Einschluß von Wacholderöl und Terpentinöl aufgelöst werden können.

In einer weiteren bevorzugten Ausführungsform handelt es sich bei dem erfindungsgemäßen Stoffgemisch um ein Nahrungsergänzungsmittel oder ein Arzneimittel, insbesondere ein natürliches, pflanzliches oder homöopathisches Nahrungsergänzungsmittel oder Arzneimittel. Dadurch stehen auch eventuelle Zusatzstoffe mit dem nichtsynthetischen Charakter der Basis im Einklang und ergeben gemeinsam ein rein natürliches Stoffgemisch.

In einer weiteren Ausführungsform ist in einem erfindungsgemäßen Stoffgemisch die Basis, d. h. in der Mischung der ätherischen Öle Wacholderöl und Terpentinöl, das Mischungsverhältnis der Öle zwischen etwa 7 zu 3 und etwa 3 zu 7, vorzugsweise zwischen etwa 6 zu 4 und etwa 4 zu 6 und besonders bevorzugt etwa 1 zu 1, gemessen an Volumsteilen. Kleinere Abweichungen des genauen Mischungsverhältnisses von 1:1 sind tolerierbar.

In einer weiteren Ausführungsform ist in einer oral einnehmbaren Kapsel oder einer anderen oral einnehmbaren Einheit eines erfindungsgemäßen Stoffgemisches zwischen etwa 10 mg und etwa 30 mg, vorzugsweise zwischen etwa 15 mg und etwa 25 mg und besonders bevorzugt etwa 20 mg Wacholderöl enthalten. Der bevorzugte Gehalt an Terpentinöl ergibt sich aus den zuvor definierten Mischungsverhältnissen. Diese vergleichsweise hohen Dosierungen haben sich im Zusammenhang mit dem Auftreten der oben beschriebenen Effekte als besonders vorteilhaft erwiesen.

In einer besonders bevorzugten Ausführungsform weist das erfindungsgemäße Stoffgemisch auf Basis von Terpentinöl und Wacholderöl des Weiteren einen oder eine Mischung mehrerer flüssiger oder gelöster Wirkstoffe auf. Diese flüssigen oder gelösten Wirkstoffe umfassen individuell nichtsynthetischen Arzneien ausgewählt aus der Gruppe Bakterien, Nosoden, Pflanzenextrakte und körpereigene Stoffe, und Wirkstoffe enthaltend Zn²⁺-lonen beziehungsweise Mg²⁺-lonen.

Als nichtsynthetische Arzneien sind Stoffe natürlichen, beispielsweise pflanzlichen Ursprungs zu verstehen, u.a. auch homöopathische Mittel. In den verwendeten Dosen sind sie vorzugsweise nicht zulassungspflichtig, jedoch alternativmedizinisch anerkannt und deshalb eingesetzt und erprobt.

Geeignete flüssige oder gelöste Wirkstoffe oder Wirkstoffgemische umfassen individuell Hepar bovis, Zink, Magnesium, Crataegus, Spigelia anthelmia, Tonsilitis-Nosode, Myosotis avensis, Veronica officinalis, Teucrium scorodonia, Pinus sylvestris, Gentiana lutea, Equisetum hiemale (HAB 34), Smifax, Scrophularia nodosa, Calcium phosphoricum, Natrium sulfuricum, Fumaria officinalis, Aranea diadema, Geranium robertanium, Ethanol, Geranium robertanium, Nasturium offcinale, Stapphylococcus-Nosode (der Staphylococcus epidermitis, Staphylococcus haemolyticus, Stapphylococcus simulans), Streptococcus haemolyticus-Nosode, Otitis media-Nosode, Tuberkulose-Nosode, Diphterie-Nosode, Malaria-Nosode, Cimicifuga racemosa, Strychnos nux vomica, Serotonin, Ubichinon, Aconitum napellus, Lachesis mutus, Bryonia, Eupatorium perfoliatum, Phosphor, Grippe-Nosode, Magnesium phosphoricum, Manganum phosphoricum, Natrium pyruvicum, Natrium oxalaceticum, Acidum citricum, Acidum cis-aconiticum, Barium oxalicum, Accidum succinicum, Acidum alpha-ketoglutaricum, Acidum succinicum, Acidum fumaricum, Acidum DL-malicum, Acidum phosphoricum, Argentum nitricum, Platin, Sepia officinalis, Valeriana officinalis, Avena sativa, Ignatia injeel forte und Acidum arsenicosum. Diese Wirkstoffe werden in weiterer Folge als Wirkstoffe der Gruppe (I) zusammengefasst.

Sollten diese Bestandteile in einem erfindungsgemäßen Stoffgemisch vorhanden sein kann sich die entstehende Produktmischung insbesondere zur Behandlung von Patienten mit leichten und schweren Infektionen jeglicher Art sowie anderen Erkältungskrankheiten eignen, insbesondere Grippe, Husten, Schnupfen, Erkältungskopfschmerzen, Sinusitis, Bronchitis oder Lungenentzündung. Dies gilt insbesondere in Kombination mit individuell ausgewählten Stoffen aus den nachfolgend beschriebenen Gruppen (XI) oder (XII).

Weitere geeignete flüssige oder gelöste Wirkstoffe oder Wirkstoffgemische umfassen individuell Viscum album, Arnica montana, Nicotiana tabacum, Hepar suis, Acidum arsenicosum, Ouabainum, Ranunculus bullbosus, Selenicereus grandiflorus, Nitroglycerinum, Kalium carbonicum, Kalmia latifolia, Spigelia anthelmia, Carbo vegetabilis, Acidum DLmalicum, Acidum sacrolacticum, Natrium oxalaceticum und Vena suis. Diese Wirkstoffe werden in weiterer Folge als Wirkstoffe der Gruppe (II) zusammengefasst.

Sollten diese Bestandteile in einem erfindungsgemäßen Stoffgemisch vorhanden sein kann sich die entstehende Produktmischung insbesondere Behandlung von Patienten mit Krankheitsbildern des Herz-Kreislaufsystems eignen. Dies gilt insbesondere in Kombination mit individuell ausgewählten Stoffen aus der Gruppe (I) und/oder mit Stoffen individuell ausgewählt aus den nachfolgend beschriebenen Gruppen (XI) oder (XII).

In einer weiteren Ausführungsform umfassen geeignete flüssige oder gelöste Wirkstoffe oder Wirkstoffgemische individuell Propolis, Histamin, Mucosa nasalis suis, Tetanus Antitoxin, Ren suis, Hepar suis, Zinkvaleriat, Apis mellifica, Apisinum, Urginea maritima, Kalum stibyltartaricum, Medulla spinalis suis, Medulla oblongata suis. Diese Wirkstoffe werden in weiterer Folge als Wirkstoffe der Gruppe (III) zusammengefasst.

Diese Wirkstoffe sollen vor allem dann in einem erfindungsgemäßen Stoffgemisch vorhanden sein, wenn das Stoffgemisch zur Behandlung von Patienten mit durch allergische Reaktion bedingten Symptomatiken, insbesondere stark geschwollenen Insektenstichen, akuten Asthmaanfällen oder spastischer Bronchitis bei Allergie eingesetzt werden soll. Dies gilt insbesondere in Kombination mit individuell ausgewählten Stoffen aus der Gruppe (I) und/oder mit Stoffen individuell ausgewählt aus den nachfolgend beschriebenen Gruppen (XI) oder (XIII).

In einer bevorzugten Ausführungsform sind in einem erfindungsgemäßen Stoffgemisch die als Basis verwendete Öl-Mischung und die flüssigen oder gelösten Wirkstoffe oder die entsprechenden Wirkstoffgemische in einem Volumenverhältnis von zwischen etwa 2 zu 8 und etwa 4 zu 6, bevorzugt von zwischen etwa 25 zu 75 und etwa 35 zu 65 und besonders bevorzugt von etwa 3 zu 7 vorhanden.

Sollte das erfindungsgemäße Stoffgemisch in Form einer Kapsel verabreicht werden, so ist es vorgesehen, dass eine der die Kapsel in einer Ausführungsform zwischen etwa 70 mg und etwa 200 mg, bevorzugt zischen etwa 90 mg und etwa 180 mg und besonders bevorzugt zwischen etwa 120 mg und etwa 150 mg einer flüssigen Komponente enthält. Diese flüssige Komponente besteht im Wesentlichen aus der als Basis verwendeten ÖI-Mischung und gegebenenfalls aus den flüssigen oder gelösten Stoffen beziehungsweise Stoffgemischen.

In einer weiteren bevorzugten Ausführungsform ist vorgesehen, dass ein erfindungsgemäßes Stoffgemisch neben der Basis aus Wacholderöl und Terpentinöl und optional den flüssigen oder gelösten Stoffen beziehungsweise Stoffgemischen des Weiteren einen oder eine Mischung mehrerer pulverförmiger Wirkstoffe aufweist. Pulverförmige Wirkstoffe umfassen nichtsynthetische, gegebenenfalls pflanzliche oder homöopathische Wirkstoffe und vorzugsweise individuell anorganische Ionen, insbesondere Calcium und Magnesium, Phosphor und/oder Schwefel und deren Verbindungen, Pflanzenextrakte, Fette, Nosoden, Bakterien oder Kohle. Der Begriff nichtsynthetische Arznei ist wie im Zusammenhang mit vorangehenden Ausführungsformen definiert.

In einer bevorzugten Ausführungsform umfassen pulverförmige Wirkstoffe individuell einen oder mehrere Wirkstoffe ausgewählt aus der Gruppe Arnica montana, Calendula officinalis, Hamamelis virginiana, Achillea millefolium, Atropa belladonna, Aconitum napellus, Mercurius solubilis Hahnemanni, Hepar sulfuris, Chamomilla recutita, Symphytum officinale, Bellis parennis, Echinacea angustifolia, Echinacea purpurea, Hypericum perforatum, Proteine, Kohlenhydrate, Calciumcarbonat, Magnesiumcarbonat, Milchzucker, Magnesiumstearat, Siliciumdioxid, mikrokristalline Cellulose, Talk, Polyvinylpryrroliden, Rhizoma Zingiberi officinali Rose, Calcium, Anamirta cocculus, Conium maculatum, Ambra grisea und Petroleum rectificatum, Arnica, Gentiana Lutea, Camomilla recucita, Juniperus cumunis und Atemisia Apsintium. Diese Wirkstoffe werden in weiterer Folge als Wirkstoffe der Gruppe (XI) zusammengefasst.

In einer weiteren Ausführungsform umfassen pulverförmige Wirkstoffe individuell einen oder mehrere Wirkstoffe ausgewählt aus der Gruppe Herba taraxati, Flos Sambucci, Herba Melissae, Herba Marubii albi, Herba Menthae crispae, Herba Gei, Radix Inulae, Fructus Anisis, Herba Agrimoniae, Rhizoma Valeriana, Semen Colae, Radix Gentianae, Cortex Grangulae, Fructus Juniperi, Flos Sambuci, Stigma Maidis, Herba Thymi, Herba Salviae, Herba Levistici, Rhizoma Velerianae, Herba Verbenae, Rhizoma Galangae, Folium Rosmarini, Rhizoma Rhei, Herba Equiseti arvensis, Herba Urticae, Herba Mentae Piperitae, Folium Menyanthis, Flos Chamomillae, Fructus Juniperi, Rhizoma Potentillae erectae, herba Centaurii, Radix Sarsaparillae, Cortex Condurango, Cortex Quercus, Alm, Cartaegus oxycantha L., Samubucs nigra L., Viola tricolor L., Citrus aurantium L. ssp aurantium, Sophora japonica L., Herba Melissae, Rhizoma Zingiberis, Herba Potentillae anserinae, Herba Marubii albi, Flos Crataegi oxycanthae, Carrageen, Herba Menthae Peperitae, Herba Cardui benedicti, Rhizoma Valerianae, Herba Violae tricoloris, Folium Juglandis, Fructus Juniperis, Herba Thymi, Herba Urticae, Fructus juniperis, Herba Fumariae, Carrageen, Herba violae odoratae, Flos trifolii pratensis, Clematis recta, Herba Scolopendri, Rhizoma valerianae, Cortex Chinchonae, Folium rosmarini, Cortex condurango, Fucus vesiculosus L. Sophora japonica L., Salvia officinalis L., Juniperus communis L., Trigonella foenum-graecum L. Linum usitatissimum L., Curcuma longa L., Chillea millefolium L., Viola tricolor L., Cola nitida (Vent) A, Chev, Argimonia eupatorai L. Cinchona succiruba Pavex Klotsch, Marsdenia cundurango Reichb. Fill., Quercus rubor L., Guajacum officinale L., Punica Granatum L., Rhizoma Gentiana Lutea L., Rhizoma Inula helenium L., Smilax aristolochiafolia Mill., Angelica archangelica L., Rhadix Alpina officinarum Hance, Pimpinella saxifrage L., Rhizoma Rheum palmatum L., Potentilla erecta L., Viola odorata L., Urtica dioica L., Viola tricolor L., Euphrasia officinalis L., Valeriana officinalis L., Flos Juglans regia L., Malva sylvestris L., Althaea officinalis L., Plantago lanceolata L., Foeniculum vulgara Mill., Matricaria chamomilla L., Phaseolus vulgatis L., Salvia officinalis L., Berberis vulgaris L., Menthax piperita L., Taraxacum officinalis Web., Valeriana officinalis L., Trigonella foenum-graecum L., Lavandula angustifolia Mill., Melissa officinalis L., Centaurium erythraea Rafin., Cnicus benedictus L., Humulus lupus L., Chondurs crispus Stackh. (Irish Moos), Citrus aurantium L. ssp aurantium, Quercus petrae Liebl., Nasturcium officinale R.Br, Taraxacum officinale Web, Primula veris L., Levisticum officinale Koch, Smilay aristolochiafolia Mill., Rhadix Imperatoria asthruthium L., Euphorbia pilulifera, Jaquin, Gallium odaratum L. Scop, Verbena officinalis L., Hamamelis virginiana L., Viola odoratum L., Teucrium scordium L., Origanum vulgare L., Euphrasia officinalis L., Crataegus oxycantha L., Hederahelix L., Equisetum arvense L., Juniperus communis L., Valeriana officinalis L., Viola tricolor L., Crataegus oxycantha L., Menthax piperita L., Matricaria chamoilla L., Sophora japonica L., Potentilla erecta Raeusch, Melissa officinalis L., Verbascumphlomoides L., Artemisia absinthum L., Menyanthis trifoliata L., Fumaria offici-nalis L., Berberis vulgaris L., Salvia officinalis L., Rhamnus frangula L., Juglan regia L. und Cnicus benedictus L. Diese Wirkstoffe werden in weiterer Folge als Wirkstoffe der Gruppe (XII) zusammengefasst.

Sollten diese Bestandteile in einem erfindungsgemäßen Stoffgemisch vorhanden sein kann sich die entstehende Produktmischung insbesondere Behandlung von Patienten mit leichten und schweren Infektionen jeglicher Art sowie anderen Erkältungskrankheiten eignen, insbesondere Grippe, Husten, Schnupfen, Erkältungskopfschmerzen, Sinusitis, Bronchitis oder Lungenentzündung, oder mit Krankheitsbildern des Herz-Kreislaufsystems eignen. Dies gilt insbesondere in Kombination mit individuell ausgewählten Stoffen aus den Gruppen (I), (II) oder (XI).

In einer weiteren Ausführungsform umfassen pulverförmige Wirkstoffe individuell einen oder mehrere Wirkstoffe ausgewählt aus der Gruppe Acidum silicicum, Histaminum muriaticum , Apis mellifica, Apis inum, Urginia, Maritima und Calium Stibyltartaricum. Diese Wirkstoffe werden in weiterer Folge als Wirkstoffe der Gruppe (XIII) zusammengefasst.

Diese Wirkstoffe sollen vor allem dann in einem erfindungsgemäßen Stoffgemisch vorhanden sein, wenn das Stoffgemisch zur Behandlung von Patienten mit durch allergische Reaktion bedingten Symptomatiken, insbesondere stark geschwollenen Insektenstichen, akuten Asthmaanfällen oder spastischer Bronchitis bei Allergie eingesetzt werden soll. Dies gilt insbesondere in Kombination mit individuell ausgewählten Stoffen aus den Gruppen (I), (III) oder (XI).

All diejenigen von den im Zusammenhang mit den vorangegangenen Ausführungsformen diskutierten Stoffe können, sofern es sich um homöopathische Stoffe handelt, in beliebigen homöopathischen Potenten eingesetzt werden, bevorzugt finden jedoch die Potenzen D1, D2, D3, D4, D6, D8, D10 und D30 Verwendung.

Für eine Dosierung, gegebenenfalls eine Kapsel eines erfindungsgemäßen Stoffgemisches ist es in einer Ausführungsform vorgesehen, dass diese zwischen etwa 200 mg und etwa 400 mg, vorzugsweise zwischen etwa 250 mg und etwa 350 mg und besonders bevorzugt etwa 300 mg des oder der pulverförmigen Wirkstoffe enthält.

Die Erfindung betrifft ferner die Verwendung eines erfindungsgemäßen Stoffgemisches zur Herstellung eines Arzneimittels oder Nahrungsergänzungsmittels, insbesondere eines nichtsynthetischen, pflanzlichen oder homöopathischen Arzneimittels. Die Therapie kann dabei als alleinige Therapie oder als unterstützende Therapie herkömmlicher schulmedizinischer oder anderer alternativmedizinischer Therapien verwendet werden. Sie soll vorzugsweise nur während der Krankheit durchgeführt werden. Dosierung und Therapiedauer sollen sich generell an der Schwere der Krankheit einstellen.

In einer bevorzugten Ausführungsform ist vorgesehen, dass im akuten Zustand der Beschwerden 4 Tage lang 4x1 Kapseln (morgens, mittags, abends, zur Nacht), danach 3 Tage lang 3x1 Kapseln (morgens, mittags, abends) und danach 2 Tage lang 2x1 Kapseln (morgens und abends) eingenommen werden sollen. In einer anderen Ausführungsform ist jedoch vorgesehen, dass die Dosis gegenüber der vorangehenden Ausführungsform reduziert werden kann, wenn die Symptome nicht allzu stark sind oder bereits frühzeitig eine Besserung eintritt. Demnach kann die Einnahme von 3 Tage lang 3x1 Kapseln (morgens, mittags, abends) und danach 2 Tage lang 2x1 Kapseln (morgens, abends) ausreichend sein. Auch kann die Einnahme von 1 bis 2 Kapseln zur Nacht bei beginnender Erkältung oder Halsweh wirksam sein. Die Eigenschaften der Kapseln und die Mengen und Verhältnisse der enthaltenen Wirkstoffe wurden im Zusammenhang mit vorangehenden Ausführungsformen diskutiert. Die resultierenden Dosen dienen als Referenz für erfolgversprechende Therapien mit Hilfe eines erfindungsgemäßen Stoffgemisches.

In einer bevorzugten Ausführungsform wird das erfindungsgemäße Stoffgemisch zur Herstellung eines oral einnehmbaren Arzneimittels verwendet. Das Arzneimittel soll in einer weiteren Ausführungsform zur Therapie von leichten und schweren Infektionen jeglicher Art sowie anderen Erkältungskrankheiten eignen, insbesondere Grippe, Husten, Schnupfen, Erkältungskopfschmerzen, Sinusitis, Bronchitis oder Lungenentzündung bestimmt sein, und in anderen Ausführungsformen zur Therapie von durch allergische Reaktion bedingten Symptomatiken wie Asthma und/oder Krankheitsbildern des Herz-Kreislaufsystems.

In einer anderen Ausführungsform soll ein auf Basis eines erfindungsgemäßen Stoffgemisches erfindungsgemäß hergestelltes Arzneimittel zum Einsatz bei grippalen Infekten, gegebenenfalls einer derartigen Erkrankung im Zusammenhang mit einer Grippe-Epidemie geeignet sein. Der vorteilhafte Einsatz wird hier besonders durch die Schelligkeit und die Stärke des Wirkungseintrittes begründet, wobei inflammationseffekte stark minimiert und die unangenehmen und potentiell bedrohlichen, starken Symptome bei akut infizierten Patienten rasch gelindert werden können.

In einer weiteren Ausführungsform ist ein erfindungsgemäßes Stoffgemisch als Arzneimittel oder Nahrungsergänzungsmittel bei der Behandlung von Miktionsstörungen bei Prostatahyperplasie, Wundheilungsproblemen, Kieferproblemen, Zahnschmerzen, Rheuma und/oder Bauchschmerzen einsetzbar. Ferner betrifft die Erfindung die Verwendung eines erfindungsgemäßen Stoffgemisches enthaltend einen oder mehrere, vorzugsweise die Gesamtheit der Wirkstoffe aus den Wirkstoffgruppen I und II und XI und XIII zur Behandlung von Patienten mit durch allergische Reaktion bedingten Symptomatiken, insbesondere stark geschwollenen Insektenstichen, akuten Asthmaanfällen oder spastischer Bronchitis bei Allergie und anderen allergisch bedingten Reaktionen oder Symptomen. Dabei soll das erfindungsgemäße Stoffgemisch alleine oder als unterstützende Therapie Anwendung finden.

Ferner betrifft die vorliegende Erfindung die Verwendung eines erfindungsgemäßen Stoffgemisches enthaltend einen oder mehrere, vorzugsweise die Gesamtheit der Wirkstoffe aus den Wirkstoffgruppen I und III und XI und XII zur Behandlung von Patienten mit Krankheitsbildern des Herz-Kreislaufsystems. Das erfindungsgemäße Stoffgemisch soll dabei als alleinige oder unterstützende Therapie zum Einsatz kommen.

Weitere Einzelheiten und Vorteile der Erfindung ergeben sich aus den nachfolgenden Ausführungsbeispielen.

### Beispiel 1: Öl-Mischung als Basis:

100 ml Oleum Juniperi e Baccarae, PH.EUR.6.0, Wacholderöl, Artikelnr. PZW 2282095 der Firma Caesar & Lorenz GmbH, D-40721 Hilden werden mit 100 ml Oleum Terebinthinae rectificat., PH.EUR.6.0, Terpentinöl vom Strandkiefer-Typ, Artikelnr. PZW 1786831 der Firma Caesar & Lorenz GmbH aus D-40721 Hilden vermengt und gut gerührt.

Hieraus resultiert die Ölmischung, welche einer erfindungsgemäßen Stoffmischung als Basis zugrunde liegt.

### Beispiel 2: Herstellung einer Mischung mehrerer flüssiger oder gelöster Wirkstoffe:

Die angegebenen Mengen der folgenden Zutaten werden gut vermischt und stellen die Basis für eine Ampullemischung dar.

| **Dosierung** | **Produkt** | **Hersteller** | |
|---|---|---|---|
| 25 Ampullen à 1,1 ml | Gripp-Heel | Biologische Heel GmbH | Heilmittel |
| 10 Ampullen à 1 ml | Hepar/Stannum II | WALA Heilmittel | GmbH |
| 10 Ampullen à 1 ml | Hepar-Magnesium Stärke D4 | Weleda AG | |
| 10 Ampullen à 1 ml | Malaria Nos. D6 - D30 | Meripharm GmbH | |
| 10 Ampullen à 1 ml | Tuberculocidinum Klebs Nos. D6 | Meripharm GmbH | |
| 10 Ampullen à 1 ml | Diphterium Nos. D30 | Meripharm GmbH | |
| 10 Ampullen à 1,1 ml | Cranolinum | Biologische Heel GmbH | Heilmittel |
| 20 Ampullen à 1,1 ml | Tonsillitis-Nosode-Injeel | Biologische Heel GmbH | Heilmittel |
| 20 Ampullen à 1,1 ml | Lymphomyosot | Biologische Heel GmbH | Heilmittel |
| 20 Ampullen à 1,1 ml | Staphylococus-Injeel | Biologische Heel GmbH | Heilmittel |
| 20 Ampullen à 1,1 ml | Streptococus haemolyticus-Injeel | Biologische Heel GmbH | Heilmittel |
| 20 Ampullen à 1,1 ml | Otitis media-Nosode-Injeel | Biologische Heel GmbH | Heilmittel |
| 20 Ampullen à 1,1 ml | Cimicifuga-Injeel forte S | Biologische Heel GmbH | Heilmittel |
| 20 Ampullen à 1,1 ml | Zitronensäurezyklus-Heel | Biologische Heel GmbH | Heilmittel |
| 20 Ampullen à 1,1 ml | Para-Benzochinon-Injeel forte | Biologische Heel GmbH | Heilmittel |
| 20 Ampullen à 1,1 ml | Acidum fumaricum-Injeel forte | Biologische Heel GmbH | Heilmittel |
| 20 Ampullen à 1,1 ml | Ubichinon-Injeel forte | Biologische Heel GmbH | Heilmittel |
| 20 Ampullen à 1,1 ml | Nux vomica-Injeel S | Biologische Heel GmbH | Heilmittel |
| 20 Ampullen à 1,1 ml | Serotonin-Injeel | Biologische Heel GmbH | Heilmittel |
| 20 Ampullen à 1,1 ml | Neuro-Injeel | Biologische Heel GmbH | Heilmittel |
| 10 Ampullen à 1,1 ml | Arsenicum Album-Injeel S | Biologische Heel GmbH | Heilmittel |
| 7 Tropfen | Bachblüten Notfalltropfen | Nelsons London | Pharmacy |

Die entstehende Mischung stellt ein Ausführungsbeispiel für die Wirkstoffmischung (I) wie im allgemeinen Teil der Patentbeschreibung definiert dar.

### Beispiel 3: Herstellung einer weiteren Mischung mehrerer flüssiger oder gelöster Wirkstoffe:

Zu einer Ampullenmischung aus Beispiel 2 werden des Weiteren folgende Zutaten hinzugefügt und das entstehende Gemisch gut vermischt.

| **Dosierung** | **Produkt** | **Hersteller** | |
|---|---|---|---|
| 10 Ampullen á 1,1ml | Cor suis compositum N | Biologische | Heilmittel |
| 10 Ampullen á 1,1ml | Arteria suis-Injeel forte | Heel GmbH Biologische | Heilmittel |
| 10 Ampullen á 1,1ml | Vena suis-Injeel | Heel GmbH Biologische | Heilmittel |
| 10 Ampullen á 1,1ml | Aorta suis-Injeel forte | Heel GmbH Biologische | Heilmittel |
| | | Heel GmbH | |

Diese Mischung stellt ein Ausführungsbeispiel für die Wirkstoffmischung (II) wie im allgemeinen Teil der Patentbeschreibung definiert dar.

### Beispiel 4: Herstellung einer weiteren Mischung mehrerer flüssiger oder gelöster Wirkstoffe:

Zusätzlich zu der Ampullenmischung aus Beispiel 2 werden des Weiteren folgende Zutaten hinzugefügt und das entstehende Gemisch gut vermischt.

| **Dosierung** | **Produkt** | **Hersteller** | |
|---|---|---|---|
| 4 g auf 100 g EtOH 96% | Propolis | | |
| 20 Ampullen à 1,1ml | Apis-Injeel S | Biologische Heel GmbH | Heilmittel |
| 20 Ampullen à 1,1ml | Histamin-Injeel | Biologische Heel GmbH | Heilmittel |
| 20 Ampullen à 1,1ml | Pollens-Injeel forte | Biologische Heel GmbH | Heilmittel |
| 20 Ampullen à 1,1ml | Mucosa nasalis suis-Injeel | Biologische Heel GmbH | Heilmittel |
| 20 Ampullen à 1,1ml | Medulla oblongata suis-Injeel | Biologische Heel GmbH | Heilmittel |
| 20 Ampullen à 1,1ml | Medulla spinalis suis-Injeel | Biologische Heel GmbH | Heilmittel |
| 20 Ampullen à 1,1ml | Tetanus antitoxin | Biologische Heel GmbH | Heilmittel |
| 20 Ampullen à 1,1ml | Ren suis-Injeel | Biologische Heel GmbH | Heilmittel |
| 20 Ampullen à 1,1ml | Hepar suis-Injeel | Biologische Heel GmbH | Heilmittel |
| 20 Ampullen à 1,1ml | Zincum valerianicum-Injeel | Biologische Heel GmbH | Heilmittel |

Diese Mischung stellt ein Ausführungsbeispiel für die Wirkstoffmischung (III) wie im allgemeinen Teil der Patentbeschreibung definiert dar.

### Beispiel 5: Herstellung einer Mischung pulverförmiger Wirkstoffe:

Die folgenden Inhaltsstoffe werden in den angegebenen Dosierungen fein zerstampft und zermahlen und anschließend gut durchmischt:

| **Dosierung** | **Produkt** | **Hersteller** | |
|---|---|---|---|
| 23 Dosen à 50 Tabletten | Traumeel S | Biologische | Heilmittel |
| | | Heel GmbH | |
| 3 Dosen à 120 Tabletten | VermiCAlite plus | Staufen Göppingen | |
| 1 Dose à 200 Tabletten | Carbo animalis | DHU | |
| (100 mg) | | | |
| 1 Dose à 200 Tabletten | Carbo vegetabilis | DHU | |
| (100 mg) | | | |
| 1,5 Tüten à 96mg | Wolle's Nr. 370 | Wolle Nature System | |
| 2 Dosen à 100 Tabletten | Vertigoheel | Biologische | Heilmittel |
| | | Heel GmbH | |
| 1 Dose | Knattwigast | Pharmazeutische | Fabrik |
| | | Kattwiga | |
| 1 Dose à 10 g | Arnica D12 Globuli | DHU | |

Diese Mischung stellt ein Ausführungsbeispiel für die Wirkstoffmischung (XI) wie im allgemeinen Teil der Patentbeschreibung definiert dar.

### Beispiel 6: Herstellung einer weiteren Mischung pulverförmiger Wirkstoffe:

Zusätzlich zur Mischung aus Beispiel 5 werden folgende weitere Inhaltsstoffe mit den Inhaltsstoffen aus Beispiel 5 vermahlen:

| **Dosierung** | **Produkt** | **Hersteller** |
|---|---|---|
| 4 Dosen | Wolle's Nr. 1781 | Wolle Nature System |
| 4 Dosen | Wolle's Nr. 1778 | Wolle Nature System |
| 4 Dosen | Wolle's Nr. 1748 | Wolle Nature System |
| 4 Dosen | Wolle's Nr. 1752 | Wolle Nature System |
| 4 Dosen | Wolle's Nr. 1706 | Wolle Nature System |
| 4 Dosen | Wolle's Nr. 1722 | Wolle Nature System |
| 4 Dosen | Wolle's Nr. 1731 | Wolle Nature System |
| 4 Dosen | Wolle's Nr. 1703 | Wolle Nature System |
| 4 Dosen | Wolle's Nr. 1784 | Wolle Nature System |
| 4 Dosen | Wolle's Nr. 1724 | Wolle Nature System |
| 4 Dosen | Wolle's Nr. 1702 | Wolle Nature System |
| 4 Dosen | Wolle's Nr. 1741 | Wolle Nature System |

Diese Mischung stellt ein Ausführungsbeispiel für die Wirkstoffmischung (XII) wie im allgemeinen Teil der Patentbeschreibung definiert dar.

### Beispiel 7: Herstellung einer weiteren Mischung pulverförmiger Wirkstoffe:

Zu der Pulvermischung aus Beispiel 5 werden folgende weitere Inhaltsstoffe mit den Inhaltsstoffen aus Beispiel 5 vermahlen:

| **Dosierung** | **Produkt** | **Hersteller** |
|---|---|---|
| 1 Dose á 100 Tabletten | KieselsäureTabletten | Cosmochema |
| (100 mg) | PZN 4400170 | |
| 1 Dose á 100 Tabletten | Histaminum Muriaticum | DHU |
| (100 mg) | | |
| 1 Dose á 100 Tabletten | Pollens | DHU |
| (100 mg) | | |
| 1 Dose á 100 Tabletten Apis mellifica | | DHU |
| (100 mg) | | |

Diese Mischung stellt ein Ausführungsbeispiel für die Wirkstoffmischung (XIII) wie im allgemeinen Teil der Patentbeschreibung definiert dar.

### Beispiel 8: Stoffgemisch zur Behandlung von Infektionen

6 ml der Öl-Mischung aus Beispiel 1 werden mit 14 ml der Ampullemischung aus Beispiel 2 und 300 mg der Pulvermischung aus Beispiel 6 vermengt und gut vermischt. Das entstehende Stoffgemisch wird in einer Weichgelatinekapsel verschweißt und dient der Einnahme durch den Patienten. Eine solche Kapsel enthält folglich grob 4 Tropfen entsprechend ca. 20 mg Wacholderöl.

### Beispiel 9: Stoffgemisch zur Behandlung von durch allergische Reaktionen bedingte Symptomatiken

6 ml der Öl-Mischung aus Beispiel 1 werden mit 14 ml der Ampullemischung aus Beispiel 3 und 300 mg der Pulvermischung aus Beispiel 7 vermengt und gut vermischt. Das entstehende Stoffgemisch wird in einer Weichgelatinekapsel verschweißt und dient der Einnahme durch den Patienten. Eine solche Kapsel enthält folglich ebenfalls grob 4 Tropfen entsprechend ca. 20 mg Wacholderöl.

### Beispiel 10: Rezeptur zur Behandlung von Krankheitsbildern des Herzkreislaufsystems

6 ml der ÖI-Mischung aus Beispiel 1 werden mit 14 ml der Ampullemischung aus Beispiel 4 und 300 mg der Pulvermischung aus Beispiel 6 vermengt und gut vermischt. Das entstehende Stoffgemisch wird in einer Weichgelatinekapsel verschweißt und dient der Einnahme durch den Patienten. Eine solche Kapsel enthält folglich ebenfalls grob 4 Tropfen entsprechend ca. 20 mg Wacholderöl.

## Patentansprüche

1. Stoffgemisch auf Basis einer Mischung von ätherischen Ölen,
**dadurch gekennzeichnet, dass**
die Öl-Mischung Wacholderöl und Terpentinöl, insbesondere Terpentinöl vom Strandkiefer-Typ enthält oder im Wesentlichen daraus besteht, und vorzugsweise dass
das Stoffgemisch des Weiteren einen oder eine Mischung mehrerer flüssiger und/oder gelöster Wirkstoffe und/oder einen oder eine Mischung mehrerer pulverförmiger Wirkstoffe beinhaltet.

2. Stoffgemisch nach Anspruch 1, wobei das Stoffgemisch zur oralen Einnahme bestimmt ist.

3. Stoffgemisch nach Anspruch 1 oder 2, wobei das Stoffgemisch portioniert in Form einer oral einnehmbaren Kapsel, vorzugsweise einer Weichgelatinekapsel vorliegt.

4. Stoffgemisch nach Anspruch 3, wobei eine Kapsel zwischen 10 und 30 mg, vorzugsweise zwischen 15 und 25 mg und besonders bevorzugt 20 mg Wacholderöl enthält.

5. Stoffgemisch nach Anspruch 3 oder 4, wobei eine Kapsel zwischen 70 und 200 mg, vorzugsweise zwischen 90 und 180 mg und besonders bevorzugt zwischen 120 und 150 mg einer flüssigen Komponente im Wesentlichen bestehend aus der Öl-Mischung und der Mischung flüssiger und/oder gelöster Wirkstoffe enthält.

6. Stoffgemisch nach einem der Ansprüche 3 bis 5, wobei eine Kapsel zwischen 200 und 400 mg, vorzugsweise zwischen 250 und 350 mg und besonders bevorzugt 300 mg der Pulvermischung enthält.

7. Stoffgemisch nach einem der vorangehenden Ansprüche, wobei es sich bei dem Stoffgemisch um ein Nahrungsergänzungsmittel oder ein Arzneimittel, insbesondere ein natürliches, pflanzliches oder homöopathisches Nahrungsergänzungsmittel oder Arzneimittel handelt.

8. Stoffgemisch nach einem der vorangehenden Ansprüche, wobei die Öl-Mischung Wacholderöl und Terpentinöl in einem Volumenverhältnis von zwischen 7:3 und 3:7, vorzugsweise von zwischen 6:4 und 4:6 und besonders bevorzugt 1:1 vorhanden sind.

9. Stoffgemisch nach einem der vorangehenden Ansprüche, wobei die flüssigen oder gelösten Wirkstoffe nichtsynthetischen Arzneien ausgewählt aus der Gruppe Bakterien, Nosoden, Pflanzenextrakte und körpereigene Stoffe, und Wirkstoffe enthaltend Zn²⁺-lonen beziehungsweise Mg²⁺-lonen umfassen.

10. Stoffgemisch nach einem der vorangehenden Ansprüche, wobei die Öl-Mischung und die flüssigen oder gelösten Wirkstoffe oder die entsprechenden Wirkstoffgemische im Verhältnis von zwischen 2:8 und 4:6, bevorzugt von zwischen 25:75 und 35:65 und besonders bevorzugt bei 3:7 vorhanden sind.

11. Stoffgemisch nach einem der vorangehenden Ansprüche, wobei die pulverförmigen Wirkstoffe anorganische Ionen, insbesondere Calcium und Magnesium, Phosphor und/oder Schwefel und deren Verbindungen, Pflanzenextrakte, Fette, Nosoden, Bakterien oder Kohle umfassen.

12. Verwendung eines Stoffgemisches nach einem der vorangehenden Ansprüche zur Herstellung eines Arzneimittels, insbesondere eines nichtsynthetischen, pflanzlichen oder homöopathischen Arzneimittels.

13. Verwendung nach Anspruch 12, wobei das Arzneimittel für die alleinige oder unterstützende orale Gabe an Patienten mit Infektionen und/oder durch allergische Reaktion bedingten Symptomatiken und/oder Krankheitsbildern des Herz-Kreislaufsystems bestimmt ist.

14. Verwendung nach Anspruch 13, wobei Infektionen Erkältungskrankheiten umfassen, insbesondere grippale Infekte, Husten, Schnupfen, Erkältungskopfschmerzen, Sinitis, Bronchitis oder Lungenentzündung.

15. Verwendung nach Anspruch 12, wobei das Arzneimittel für die alleinige oder unterstützende orale Gabe an Patienten mit einem oder mehreren Symptomen ausgewählt aus der Gruppe Miktionsstörungen bei Prostatahyperplasie, Wundheilungsproblemen, Kieferproblemen, Zahnschmerzen, Rheuma und Bauchschmerzen bestimmt ist.
